**Europäisches Patentamt**

**European Patent Office**

**Office européen des brevets**

(19)

(11) Veröffentlichungsnummer: **0 118 601**
**B1**

(12) **EUROPÄISCHE PATENTSCHRIFT**

(45) Veröffentlichungstag der Patentschrift:
30.07.86

(21) Anmeldenummer: 83111289.1

(22) Anmeldetag: 11.11.83

(51) Int. Cl.⁴: **A 61 L 2/26, C 12 Q 1/22,
A 61 L 2/02**

(54) Vorrichtung zur Sterilitätsprüfung von Fluiden.

(30) Priorität: 15.02.83 FR 8302391

(43) Veröffentlichungstag der Anmeldung:
19.09.84 Patentblatt 84/38

(45) Bekanntmachung des Hinweises auf die Patenterteilung: 30.07.86 Patentblatt 86/31

(84) Benannte Vertragsstaaten:
AT BE CH DE GB IT LI NL SE

(56) Entgegenhaltungen:
GB-A- 2 015 730
US-A- 3 970 084
US-A- 4 014 797
US-A- 4 292 405
US-A- 4 351 900

(73) Patentinhaber: MILLIPORE S.A.
43, Avenue de L'Europe
F-78 140 Velizy-Villacoublay (FR)

(72) Erfinder: Lemonnier, Jean
63, Allée de la Meute
F-78110 Le Vesinet (FR)

(74) Vertreter: Henkel, Feiler, Hänzel & Partner
Möhlstrasse 37
D-8000 München 80 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 118 601 B1

## Beschreibung

Die Erfindung betrifft eine Vorrichtung zur Sterilitätsprüfung von Fluiden, insbesondere von Antibiotika enthaltenden Fluiden, mit mindestens einem hermetisch geschlossenen Prüfbehälter aus transparentem Kunststoff, der über Schlauchverbindungen und eine Kanüle steril an einen Probenbehälter und/oder an weitere Flüssigkeitsbehälter anschließbar ist, wobei jeder Prüfbehälter aus einem einstückigen napfförmigen Oberteil mit einem Einlaßstutzen, einem Lufteinlaß und einem darin integrierten Luftfilter in seinem oberen Bereich sowie aus einem einstückigen Unterteil mit einem Dränageglied, einem mechanischen Träger für einen ebenen, mikroporösen Prüffilter und einem Auslaßstutzen besteht, und wobei beide Behälterteile an ihren Rändern miteinander druckdicht verschweißt sind und der Prüffilter zwischen dem unteren Rand des zylindrischen Oberteils und dem oberen Rand des zylindrischen Unterteils unlösbar und gasdicht befestigt ist.

In der medizinischen Technik und auf anderen technischen Gebieten besteht häufig eine unabdingbare Forderung, daß die eingesetzten Gebrauchsgegenstände und die verwendeten Fluide absolut steril sind. Dies gilt in besonderem Maße beispielsweise für Infusionslösungen, die Patienten direkt intravenös injiziert werden, sowie für die dabei verwendeten Arbeitsgerätschaften. Aufgrund der außerordentlichen Bedeutung der praktisch absoluten Sterilität von Arbeitsgerätschaften und Fluiden müssen regelmäßig Sterilitätsprüfungen vorgenommen werden, bei deren Durchführung strenge gesetzliche Bestimmungen einzuhalten sind (in USA z. B. die Pharmacopeia). Bei Sterilitätsprüfungen von Antibiotika ergaben sich bisher regelmäßig besondere, durch die spezifischen Eigenschaften dieser Prüfmedien bedingte Schwierigkeiten, die nur durch eine besonders hohe Sorgfalt bei der Versuchsdurchführung sowie durch eine entsprechend größere Anzahl von Einzelprüfungen überwunden werden konnten.

Bei einer in der Vergangenheit weitverbreiteten Prüfmethode wurde die auf Sterilität zu prüfende Flüssigkeit in mehrteiligen Prüfbehältern aus durchsichtigem Kunststoff filtriert (vgl. Druckschrift MRP-4 Millipore «Sterilitätsprüfung» Sept. 1968). Die Prüfbehälter bestehen aus einem zylindrischen Oberteil mit aufsetzbarem Deckel, in dessen Stirnwand mehrere Anschlußstutzen für Schläuche und für eine Luftfilter-Patrone angeordnet sind. Ein Behälterunterteil aus Kunststoff weist einen ebenen gerippten Filterträger für einen mikroporösen Membranfilter und an seinem Außenumfang ein Gewinde auf, auf das das eingezogene untere Ende des Oberteils aufgeschraubt werden kann. Durch dieses Aufschrauben des Oberteils wird der mikroporöse ebene Membranfilter zwischen den Träger und einer eine O-Dichtung aus Gummi aufweisenden Ringschulter am Oberteil fest eingespannt. Das zu prüfende Fluid wird unter sterilen Bedingungen bei abgehobenem Deckel in den Oberteil des Behälters von Hand eingefüllt und strömt nach Anlegen eines Unterdruckes durch das mikroporöse Membranfilter hindurch. Nach dem Filtrieren wird unter ebenfalls sterilen Bedingungen das Oberteil abgeschraubt, der zentrale Teil des Membranfilters herausgestanzt, in eine Kulturflasche eingebracht und darin unter vorgegebenen Temperaturen bebrütet. Diese Prüfmethode ist jedoch außerordentlich aufwendig, da sie in ihren verschiedenen Stadien stets sterile Umgebungsbedingungen voraussetzt und verschiedene Eingriffe von außen verlangt, wie z. B. das Aufschrauben der Behälterteile, die Entnahme des Membranfilters, seine Zerschneidung und Überführung in die Bebrütungsgefäße. Darüber hinaus weist der Prüfbehälter Fugen zwischen seinen zusammenmontierten Einzelteilen auf, durch die Umgebungsluft in das Behälterinnere gelangen und Sekundär-Kontaminationen herbeiführen kann. Zur besonders kritischen Prüfung von Antibiotika haben die Membranfilter einen hydrophoben Rand, durch den verhindert werden soll, daß sich Reste der antibiotischen Prüfflüssigkeit am äußersten Filterrand absetzen und während der Bebrütungszeit die sich möglicherweise bildenden Bakterien- und Pilzkolonien zerstören.

In neuerer Zeit hat sich eine Vorrichtung zur Sterilitätsprüfung der eingangs genannten Gattung bei den Anwendern in erheblichem Umfange durchgesetzt (vgl. DE-OS-2 549 835), bei der zwei oder drei hermetisch geschlossene Prüfbehälter aus durchsichtigem Kunststoff über angeschweißte Schlauchverbindungen über einem Stromteiler mit einer Kanüle steril verbunden sind. Jeder Prüfbehälter besteht aus einem einstückigen napfförmigen Oberteil, in dessen Stirnwand ein mit dem Schlauch fest verbundener Einlaßstutzen sowie ein Lufteinlaß mit einem darin unlösbar integrierten Luftfilter angeordnet sind. Der Behälteroberteil weist am unteren Rand seiner zylindrischen Wand einen Ringflansch auf, über den er mit dem oberen Ringflansch eines Behälterunterteils dauerhaft und gasdicht verschweißt ist. Der Behälterunterteil ist ebenfalls einstückig aus einem durchsichtigen Kunststoff ausgeführt und besteht im wesentlichen aus einem konischen Sammeltrichter, der an seiner Oberseite eine Vielzahl von winkelversetzten Radialrippen als Träger für den ebenen Membranfilter aufweist und der in einen zentralen Auslaßstutzen ausmündet. Der wesentliche Vorteil dieser bekannten Prüfvorrichtung gegenüber älteren Ausführungen liegt in dem in sich druckdicht geschlossenen und damit von vornherein sterilen System, bestehend aus mehreren der hermetisch geschlossenen Prüfbehälter, den an die einzelnen Behälter angeschweißten Schlauchverbindungen, dem Stromteiler und der Kanüle. Da Luft nur über den in die Deckwand eingeschweißten mikroporösen Luftfilter in das Innere der jeweiligen Behälter gelangen kann, sind Verfälschungen der Prüfergebnisse durch Sekundär-Kontaminationen praktisch ausgeschlossen. Ferner besteht mit dieser Vorrichtung erstmals die Möglichkeit einer Druckfiltration, bei welcher die Flüssigkeiten nicht durch Anlegen eines Vakuums an den zentralen Auslaufstutzen, sondern durch Einführen der Flüssigkeiten unter einem Überdruck von ca. 2 bar in die Behälter filtriert werden. Neben der Sicherstellung einer praktisch absoluten Sterilität der Versuchsdurchführung auch in nicht-sterilen Räumen ergibt sich durch die erstmals mögliche Druckfiltration eine erhebliche Beschleunigung des Prüfungsablaufes. Nach dem gleichzeitig in mehreren Prüfbehältern erfolgenden Filtrieren einer vorgegebenen Probenmenge an Prüfflüssigkeit werden alle Behälter durch gleichzeitiges Einleiten einer sterilen Prüfflüssigkeit von Ansätzen und Resten der Prüfflüssigkeit gereinigt. Nach dem Spülvorgang werden die einzelnen Behälter mit verschiedenen Prüfmedien gefüllt, was auf einfache Weise dadurch geschieht, daß die Kanüle in den Septum-Verschluß eines Medienbehälters eingestochen wird und die vom Stromteiler zu den einzelnen Behältern führenden Schlauchverbindungen teilweise durch übliche Schlauchklemmen abgesperrt werden, so daß das Medium nur in den dafür vorgesehenen Prüfbehälter fließen kann. Die mit den Prüfmedien gefüllten Prüfbehälter werden anschließend eine vorgegebene Zeit lang bei bestimmten Temperaturen bebrütet, wobei der zentrale Auslaßstutzen durch Aufstülpen einer Silikongummi-Kappe hermetisch verschlossen ist und ein Stück des abgeschnittenen Einlaßschlauches auf den Lufteinlaßstutzen aufgestülpt ist, was ebenfalls einen hermetisch dichten Abschluß ergibt.

Mit dieser bekannten Prüfvorrichtung werden bei einer Vielzahl von Prüfmedien bei einfachster und schneller Versuchsdurchführung ausgezeichnete Ergebnisse erzielt. Nach wie vor problematisch ist jedoch die Prüfung von antibiotische Substanzen enthaltenden Flüssigkeiten. Da nämlich die Membranfilter an ihrem äußersten Rand entweder nur zwischen dem Behälterober- und -unterteil radial innerhalb der Schweißverbindung eingeklemmt sind oder aber diese Schweißverbindung durch Materialfluß das Filtermaterial vollständig durchdrungen und damit beschädigt hat, setzen sich geringe Mengen an Antibiotika in dem Verbindungsbereich des Filterrandes fest, die auch durch wiederholtes Spülen nicht mehr ausgewaschen werden können. Während der sich über längere Zeiträume erstreckenden Bebrütung können diese geringen Reste an antibiotischen Substanzen beispielsweise durch Diffusion in den zentralen Teil der Filtermembran wandern und die sich ggf. bildenden Bakterien- bzw. Pilzkulturen angreifen, was eine Verfälschung der Prüfergebnisse zur Folge hat. Zur Vermeidung dieser in der Praxis wesentlichen Probleme werden zur Prüfung von Antibiotika und vergleichbar kritischen Substanzen Vorrichtungen verwendet, bei denen die Membranfilter einen hydrophoben relativ breiten Rand und einen hydrophilen zentralen Bereich haben. Die Herstellung derartiger scheibenförmiger Filtermembranen ist jedoch langwierig und aufwendig, da ihr Rand nur durch langwierige Diffusionsprozesse hydrophob gemacht werden kann. Da ferner der hydrophobe Randbereich praktisch flüssigkeitsundurchlässig und somit an dem Filtriervorgang nicht beteiligt ist, verringern sich die Filtrierleistungen auch bei Anwendung der Druckfiltration.

Darüber hinaus ergibt sich auch ferner der Nachteil, daß durch den hydrophilen Membranrand Sauerstoff von der Ablaufseite her hindurchdiffundieren und damit in die Flüssigkeit oberhalb der Filtermembran gelangen kann, was bei Prüfungen auf das Vorhandensein bestimmter Bakterien zu einer Verfälschung der Prüfergebnisse führen kann. Bei der Prüfung von Antibiotika ist es ferner unbedingt notwendig, daß die Filtermembran völlig eben und spannungsfrei auf den Rippen des Filterträgers aufliegt. Die Befestigung der Membranfilter an den unteren Rändern der Behälterteile durch direktes Einsiegeln bzw. Einschweißen führt aufgrund der örtlich hohen Temperaturen zu Spannungen und damit zu wellenförmigen Verwerfungen der Filtermembranen. In der Praxis werden diese Verformungen dadurch vermieden, daß die Filtermembranen eine ausreichende Eigensteifigkeit haben, was einmal durch Einlagern von formsteifen Fasern in die Filtermaterialien und zum anderen durch eine ausreichende Membrandicke von 250 $\mu$m und mehr erreicht wird. Zwar werden in der Literatur geringere Membrandicken von z. B. 150 $\mu$m als filtertechnisch vorteilhafter genannt, in der Praxis können derartig dünne Filtermembranen jedoch wegen ihrer geringeren Formsteifigkeit nicht eingesetzt werden.

In der GB-A-2 015 730, insb. Spalte 2 ist ferner eine ähnliche, dem gleichen Zweck dienende Vorrichtung beschrieben, bei der ein Membranfilter vorgesehen ist, dessen Rand mit dem Geräteteil ultraschallverschweißt sein kann.

Wie ersichtlich, hängen die vorstehend genannten Probleme, die sich insbesondere bei der Filtration von Flüssigkeiten mit antibiotischen Substanzen ergeben, mit der Art der Befestigung und Verbindung der Filtermembran mit den beiden Behälterteilen zusammen.

Aufgabe der Erfindung ist es, eine Vorrichtung zur Sterilitätsprüfung von Fluiden, insbesondere von Antibiotika enthaltenden Flüssigkeiten, zu schaffen, die unter Vermeidung der vorstehend genannten Nachteile höhere Durchsatz- bzw. Filtrierleistungen bei vereinfachtem Aufbau ermöglicht und bei der die Ursachen für Fehler bei der Durchführung der Sterilitätsprüfungen vermindert sind.

Der Lösung dieser Erfindungsaufgabe liegt erfindungsgemäß die Erkenntnis zugrunde, daß der scheibenförmige Membranfilter aus mikroporösem Zellulose-Fasermaterial nur mit den beiden Oberflächen seines Außenrandes jeweils mit dem Behälterunterteil und dem Behälteroberteil mittels einer Kunststoff-Schweißverbindung durchgehend fest verbunden wird, ohne daß das Filtermaterial selbst zerstört würde oder der schmelzflüssige Kunststoff das poröse Filtermaterial durchdringt. Dabei muß sichergestellt sein, daß sich zwischen Membranoberfläche und angrenzendem Kunststoffteil weder Fugen noch Spalte bilden, noch daß schmelzflüssiger Kunststoff beim Schweißvorgang über die Innenwand des einen oder anderen Behälterteils hinaus in das Behälterinnere vordringt, damit sicher vermieden wird, daß sich geringe Mengen an Antibiotika in diesen kritischen Eckbereichen ablagern bzw. festsetzen können. Somit zeichnet sich die Prüfvorrichtung gemäß der Erfindung dadurch aus, daß der scheibenförmige Prüffilter vollständig aus einer hydrophilen Membran besteht und daß jede der beiden Oberflächen des Membranfilterrandes mit dem jeweiligen Behälterteil durch eine gesonderte Schweißverbindung druckdicht verbunden ist, die mit der Behälterinnenwand bündig abschließt.

Eine in dieser Weise ausgebildete Einsiegelung der Filtermembran mit den beiden Behälterteilen, die radial außerhalb der Filtermembran im Bereich ihrer Ringflansche direkt miteinander verschweißt sind, erlaubt die Verwendung von durchgehend hydrophilen Filtermembranen ohne den bisher notwendigen hydrophoben Randbereich, was die angestrebte konstruktive Vereinfachung der Prüfvorrichtung ergibt. Da nunmehr die gesamte Filtermembran am Filtriervorgang teilnimmt, werden bei gleichen Behälterabmessungen und Prüfbedingungen die Durchsatz- bzw. Filtrierleistungen gegenüber gattungsgleichen Vorrichtungen um ca. 40 % gesteigert. Die zur Herstellung des bisher notwendigen hydrophoben Randbereiches erforderlichen Behandlungsvorgänge fallen weg und die scheibenförmigen Membranfilter können aus Bandmaterial automatisch ausgestanzt werden, was eine wesentliche Vereinfachung des Herstellungsprozesses der Prüfbehälter ermöglicht. Da sich die jeweiligen Schweißverbindungen nur auf die Oberflächen des Filtermembranrandes beschränken und die schmelzflüssigen Kunststoffmaterialien der Behälterteile praktisch nicht in die Poren der Membran eindringen, sind die thermischen Beanspruchungen des Filtermaterials bei den Schweiß- bzw. Versiegelungsvorgängen gering, so daß die bisher häufig auftretenden wellenförmigen Verwerfungen der eingesiegelten Filtermembran vermieden werden. Dies eröffnet die bisher praktisch nicht möglichen Anwendungen von dünneren Filtermembranen ohne Faserversteifung, was einen verbesserten Flüssigkeitsdurchfluß und eine geringere Flüssigkeitsabsorption der Prüfflüssigkeit im Filter selbst zur Folge hat.

Bei einer hinsichtlich ihrer Herstellung und ihrer Dichtigkeit besonders zweckmäßigen Ausgestaltung der Erfindung ist die eine Oberfläche des Membranrandes mit dem zugeordneten Behälterrand durch eine direkte Wärme- und Druckeinwirkung verbunden, ohne daß nennenswerte Fließerscheinungen des Behältermaterials eintreten. Die zweite Oberfläche des Membranrandes ist mit dem zugeordneten Behälterteil durch einen Schmelzfluß an Behältermaterial verbunden, der von radial außerhalb der Membran in den Spalt zwischen der Membranfläche und der Behälterteilfläche eingeflossen ist. Während somit die Verbindung der ersten Oberfläche des Behälterrandes mit dem zugeordneten Behälterteil eher einer Klebeverbindung ähnelt, bei welcher der Membranfilterrand in den schmelzflüssig gemachten Kunststoff eingedrückt ist, handelt es sich bei der Verbindung der zweiten Oberfläche des Behälterrandes mit dem an sie angrenzenden Behälterteil eher um eine Schweißverbindung, bei welcher schmelzflüssiges Material von außen in den Verbindungsspalt eingebracht worden ist.

Zur Erzielung dieses Schmelzflusses ist zweckmäßig an dem ggf. als Ringflansch ausgebildeten Verbindungsrand des einen Behälterteils eine keilförmige Ringwulst radial außerhalb der scheibenförmigen Filtermembran angeformt, die beim Schweißvorgang den Schmelzfluß in den Spalt hineinliefert. Dabei ist erfindungsgemäß die Anordnung, Ausbildung und Größe dieser Ringwulst so bemessen, daß die in den Spalt einfließende Kunststoffmenge ausreicht, um eine wirksame Haftverbindung über den gesamten Filterrand bis zur Innenwand des Behälterteils zu gewährleisten, und zum anderen auch eine sichere und druckdichte Materialverschmelzung radial außerhalb des Membranfilters zwischen den beiden Behälterteilen sicherzustellen.

Um die dünneren, vollständig hydrophilen Filtermembranen mechanisch sicher abzustützen und gleichzeitig Kontaminationen von der der Umgebungsatmosphäre ausgesetzten Filterunterseite her zu vermeiden, besteht gemäß einer zweckmäßigen Ausgestaltung der Erfindung die Dränage im Behälterunterteil aus einem Ringkanal unmittelbar an der Innenwand, von dem eine Vielzahl von schmalen, durch Tragrippen begrenzten kurzen Ringkanälen ausgehen, die in Umfangskanäle im Innenbereich des Filterträgers ausmünden. Durch diese Ausgestaltung wird erreicht, daß die im unmittelbaren Wandbereich durch die Filtermembran hindurchtretenden Flüssigkeitsmengen direkt und auf kürzestem Weg in Richtung des Ablaufstutzens abfließen und sich nicht in diesen Bereichen ablagern können. Dies ist insbesondere bei Anwendung der Druckfiltration wichtig, weil ja nur der Innenraum des Behälteroberteils unter dem Überdruck steht und im Dränagesystem unterhalb der Filtermembran Atmosphärendruck herrscht, der keine Förderwirkung ausüben kann.

Bei einem Verfahren zur Herstellung von Behältern für die erfindungsgemäßen Prüfvorrichtungen werden die beiden Behälterteile gesondert in Spritzgußtechnik hergestellt, der Luftfilter einschließlich seiner beidseitigen mechanischen Träger in den Behälteroberteil eingeschweißt und die Filtermembran zwischen den beiden Behälterteilen an deren Rändern befestigt. Hierzu wird erfindungsgemäß die Filtermembran über dem Rand eines Behälterteils positioniert und ihre diesem Behälterteil zuweisende Oberfläche wird durch Zufuhr von Wärme durch die Membran hindurch und Aufbringen eines vorbestimmten Andruckes mit dem Behälterrand verschweißt. In einem anschließenden Arbeitsvorgang wird der andere Behälterteil mit seinem Rand mit der anderen Oberfläche der Membran durch Erzeugung eines nach radial einwärts gerichteten Kunststoff-Schmelzflusses sowie gleichzeitig mit dem ersten Behälterteil verschweißt. Zum Verschweißen des zweiten Behälterteils mit der Oberfläche des Membranrandes und mit dem ersten Behälterteil kann eine an einem der Behälterteile angeformte Ringwulst bis zum Aufschmelzen erhitzt werden, so daß beim Zusammenbringen der beiden Behälterteile der erzeugte Schmelzfluß in den Spalt zwischen der Oberfläche der Filtermembran und dem Behälterteil eindringt und diesen vollständig ausfüllt.

Besonders zweckmäßig ist eine Herstellung der Behälter, bei welcher die einzelnen Vorgänge automatisiert sind und das Filtermaterial in Form eines Bandes taktweise abgezogen sowie über die entsprechend positionierten vorgefertigten ersten Behälterteile transportiert wird. Aus dem Band können die kreisförmigen Filterscheiben entweder vor oder nach der Durchführung der ersten Schweißverbindung ausgestanzt werden, woraufhin dann die zweite Schweißverbindung durch Aufschmelzen des Ringwulstes und Erzeugen eines radialen Kunststoff-Schmelzflusses erfolgt.

Im folgenden wird ein Ausführungsbeispiel der Erfindung anhand der Zeichnung im einzelnen beschrieben. Es zeigen:

Figur 1 eine erfindungsgemäße Vorrichtung zur Sterilitätsprüfung mit zwei im Axialschnitt dargestellten Prüfbehältern;

Figuren 2, 3 zwei aufeinanderfolgende Stadien der Herstellung einer Verbindung zwischen den beiden Behälterteilen in vergrößertem Ausschnitt;

Figur 4 eine Draufsicht auf den Behälterunterteil mit dem Membranträger vor dem Zusammenbau beider Behälterteile;

Figuren 5a bis 5c Axialschnitte durch den Behälterunterteil in den Schnittlinien A, B bzw. C in Fig. 4.

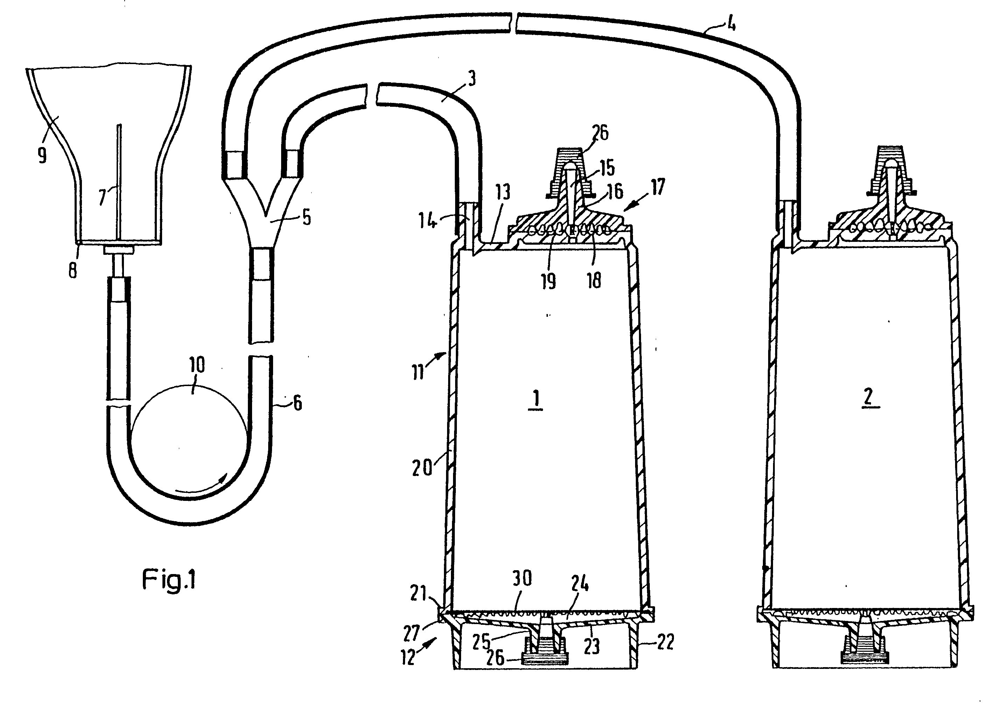

Die in Fig. 1 dargestellte Vorrichtung zur Durchführung von Sterilitätsprüfungen besteht aus zwei in sich geschlossenen Prüfbehältern 1, 2 aus durchsichtigem Kunststoff, die über je einen Schlauch 3, 4 an einen Stromteiler 5 angeschlossen sind. Dieser Stromteiler 5 ist über einen gemeinsamen Schlauch 6 mit einer Kanüle 7 verbunden, die durch einen Deckel 8 in das Innere eines mit Prüfflüssigkeit gefüllten Gefäßes 9 eingestochen worden ist. Die Flüssigkeit wird aus dem Gefäß 9 mittels einer Schlauchpumpe 10 über die Schläuche und den Verteiler in die beiden Prüfgefäße 1, 2 gleichzeitig unter einem Druck von etwa 2 bar gefördert. Um die Prüfbehälter 1, 2 einzeln mit unterschiedlichen Flüssigkeiten füllen zu können, kann entweder der Stromteiler 5 als Mehrwegehahn ausgebildet sein oder die Schläuche 3, 4 können durch übliche Klemmen wahlweise gesperrt werden.

Jeder Behälter 1, 2 besteht aus einem einstückigen Oberteil 11 und einem ebenfalls einstückigen Unterteil 12, die miteinander fest und druckdicht verschweißt sind. Der Oberteil 11 ist napfförmig ausgebildet und weist in seiner oberen Stirnwand 13 einen angeformten Einlaßstutzen 14 sowie einen Lufteinlaß 15 auf, welcher im dargestellten Zustand durch eine Silikongummi-Kappe 16 hermetisch abgedeckt ist. Der Lufteinlaß 15 bildet einen Kanal in dem Kunststoffdeckel 16 eines Luftfilters 17, dessen mikroporöse Filtermembran 18 auf einem als Scheibenrost 19 ausgebildeten Teil der Stirnwand 13 einerseits und nach oben an der gerippten Unterseite des Filterdeckels 16 abgestützt ist. Der Filterdeckel 16 ist an seinem Umfangsrand mit der Behälterstirnwand 13 gasdicht verschweißt, so daß bei abgenommener Gummikappe Luft nur über die mikroporöse Filtermembran 18 in bzw. aus dem Behälterinneren strömen kann. Am unteren Ende der leicht konischen Zylinderwand 20 des Oberteils 11 ist ein Ringflansch 21 angeformt. Der Behälteroberteil 11 besteht aus einem glasklaren Kunststoff, was die Betrachtung von Färbungen oder Trübungen der eingeschlossenen Flüssigkeiten ermöglicht.

Der einstückige Behälterunterteil 12 aus Kunststoff weist einen etwa zylindrischen Umfangsrand 22, ein Dränageglied in Form einer Trichterwand 23 mit Rippen 24 sowie einen zentralen Auslaßstutzen 25 auf, der im dargestellten Betriebszustand durch eine Gummikappe 26 abgedeckt ist. Während des Filtrierens sind die Gummikappen 26 selbstverständlich von den zentralen Auslaßstutzen 25 der beiden Behälter 1, 2 entfernt. Der Behälterunterteil 12 weist an seinem oberen Ende einen Ringflansch 27 zur dauerhaften Schweißverbindung mit dem Ringflansch 21 des Behälteroberteils 11 auf. Auf den Tragrippen 24 des Behälterunterteils 12 liegt eine Filtermembran 30 eben auf, die eine Wandstärke von etwa 150 μm hat und aus einem hydrophilen Zellulosematerial besteht. Die Porengröße dieser mikroporösen Filtermembran ist vorgeschrieben und liegt bei 0,45 ± 0,02 μm.

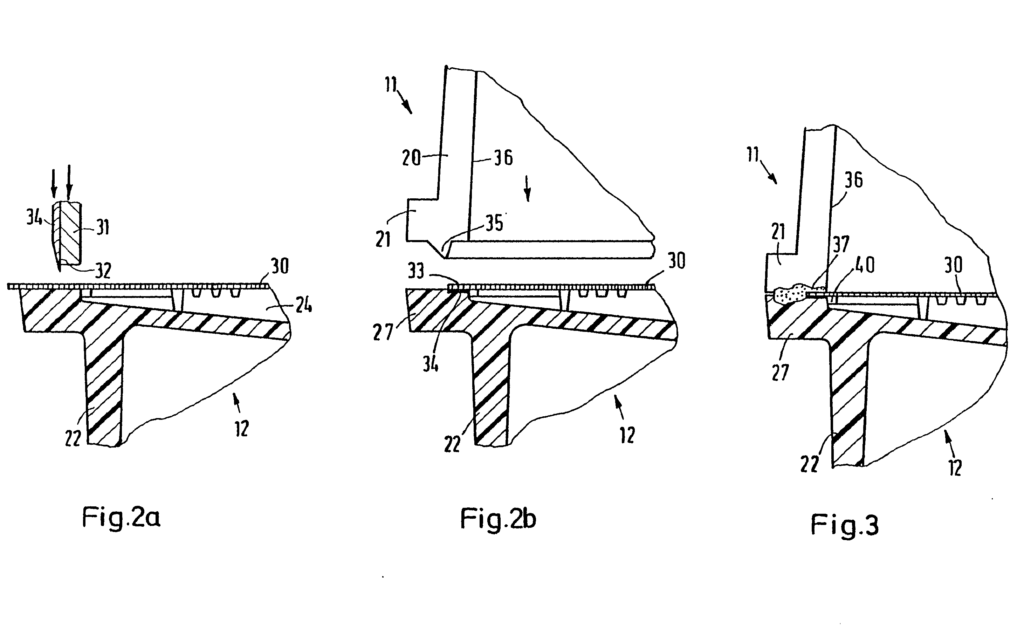

Die erfindungswesentliche Befestigung der Membran 30 im Behälter 1 bzw. 2 wird im folgenden anhand der Fig. 2a, 2b und 3 beschrieben.

Gemäß Fig. 2a ist die Filtermembran 30 in Form eines Bandes oder Streifens von einer — nicht dargestellten — Rolle taktweise über die in bestimmten Positionen festgelegten Behälterunterteile unter einer gewissen Vorspannung gezogen worden, welche eine genau ebene Ausrichtung des Bandes gewährleistet. Ein hohlzylindrisches Schweißwerkzeug 31 wird mittels nicht dargestellter Einrichtungen auf eine sehr genau festgelegte Temperatur seiner unteren Arbeitsfläche 32 gebracht und in Richtung des Pfeiles vorgeschoben. Die untere ringzylindrische Arbeitsfläche 32 des Werkzeuges 31 ist so bemessen, daß sie genau der Breite des einzuschweißenden Membranfilterrandes 33 entspricht. Das erhitzte Werkzeug 31 drückt mit seiner Arbeitsfläche 32 in einem ringförmigen Abschnitt auf die Filtermembran 30, wobei sich das Kunststoffmaterial unterhalb dieses ringförmigen Membranabschnittes auf eine vorgegebene Temperatur erwärmt und fließfähig wird. Durch den gleichzeitig wirkenden Andruck des Membranabschnittes erfolgt auf diese Weise eine feste Haftverbindung zwischen dem ringförmigen Membranabschnitt und dem Kunststoffmaterial, die in Fig. 2b als breiter Strich unterhalb des Randes 33 dargestellt ist. Nach Herstellen dieser Klebe- bzw. Schweißverbindung wird ein unmittelbar radial außerhalb des hohlzylindrischen Schweißwerkzeuges 31 vertikal verschiebbar angeordnetes Ringmesser 34 nach unten verfahren, das aus der bandförmigen Filtermembran 30 unmittelbar außerhalb der hergestellten Schweißverbindung 34 das scheibenförmige Membranfilter herausschneidet. Durch diesen Vorgang ist einmal gewährleistet, daß das Membranfilter 30 genau eben auf den Tragrippen 24 während des Schweißvorganges aufliegt, und daß es durch den sich anschließenden Schneidvorgang nicht verrutschen kann.

Nach den vorstehend beschriebenen Bearbeitungsvorgängen wird das Behälterunterteil 12 zusammen mit der aufgeschweißten Filtermembran 30 in die Position gemäß Fig. 2b gebracht, in welcher es mit dem Behälteroberteil verschweißt wird. Wie ersichtlich, ist an der Unterseite des Behälteroberteils eine keilförmige Ringwulst 35 angeformt, deren Größe und Anordnung für die Herstellung der erfindungsgemäßen Schweißverbindung von besonderer Bedeutung ist. Wie ersichtlich, befindet sich die Spitze dieser keilförmigen Ringwulst geringfügig außerhalb der Umfangskante des Filtermembran-Randes 33. Dieser Ringwulst 35 wird auf geeignete Weise, z. B. durch Ultraschall, auf die Schmelztemperatur des Kunststoffmaterials erwärmt und das Behälteroberteil 11 wird in Richtung des Pfeiles bei 36 nach unten bewegt. Dabei trifft die erwärmte Spitze des Ringwulstes 35 auf das Kunststoffmaterial des Behälterunterteils, und zwar unmittelbar neben der Außenkante des Randes 33. Bei einer weiteren Vorschubbewegung des Behälteroberteils 11 beginnt ein Teil des Kunststoffmaterials des Ringwulstes 35 auf der Oberfläche des Membranfilterrandes 33 nach radial innen zu fließen und bei anhaltender Zufuhr von Wärmeenergie fließt ein weiterer Teil des Ringwulst-Materials nach radial außen und verbindet sich mit dem Kunststoffmaterial des Behälterunterteils 12 zu einer druckdichten festen Schweißverbindung. Die Anordnung des Ringwulstes 35 an der Unterseite des Behälteroberteils ist so getroffen, daß bei der Zusammenbewegung der beiden Behälterteile 11 und 12 die Scheitellinie des Ringwulstes radial außerhalb der Filtermembran 30 auf die Oberfläche des Ringflansches 27 des Behälterteils 12 auftrifft, wodurch übermäßige mechanische Druckbeanspruchungen des Membranfilterrandes 33 vermieden werden. Die Größe und Querschnittsform des Ringwulstes 35 sind erfindungsgemäß so gewählt, daß ein ausreichender Schmelzfluß bis genau zur inneren Wandfläche 36 der Behälterwand 20 sichergestellt ist und gleichzeitig ein Aufschmelzen der Kunststoffmaterialien beider Behälterteile 11 und 12 radial außerhalb des Filtermembran-Randes 33 zum Erhalt einer hochfesten und gasdichten Schweißverbindung gewährleistet wird. Dieser Zustand ist in Fig. 3 dargestellt. Wesentlich bei der Herstellung der in der vorstehenden Weise beschriebenen Verbindung ist es, daß der Rand 33 der Filtermembran 30 nicht von den Kunststoffmaterialien durchtränkt ist, sondern daß die vorzugsweise querverlaufenden, sehr feinen Filterporen nur an ihren Öffnungsenden durch die Kunststoffmaterialien festhaftend verschlossen sind. Auf diese Weise werden höhere mechanische Druckbeanspruchungen des empfindlichen Filtermaterials vermieden, die Ursache von feinen Filterrissen sein könnten. Die genaue Anordnung und Bemessung des Ringwulstes 35 bewirkt, daß in der Ecke zwischen der inneren Wandfläche 36 und der Oberfläche der Filtermembran 30 weder Spalte noch Materialansätze vorhanden sind, an denen sich möglicherweise geringe Mengen an Prüfflüssigkeit absetzen können, die auch durch wiederholtes Spülen nicht zu entfernen wären. Darüber hinaus wird durch den zweistufigen Schweißvorgang gemäß Fig. 2a, 2b sichergestellt, daß die Filtermembran 30 völlig eben und spannungsfrei auf den Tragrippen 24 des Behälterunterteils 12 aufliegt. Da der Rand 33 der Filtermembranen keinen größeren thermischen und mechanischen Beanspruchungen bei einem dieser beiden Schweißvorgänge ausgesetzt wird, werden Verwerfungen und Welligkeiten der Filtermembran 30 vermieden, welche bei Anwendung der herkömmlichen Verbindungstechnik insbesondere bei Verwendung von Membranfiltern geringer Steifigkeit auftraten.

Dementsprechend können wesentlich dünnere Filtermembranen verwendet werden, was insbesondere bei der Durchführung der Spülvorgänge nach dem Filtrieren wesentlich ist, weil bekannterweise dünnere Filter auch entsprechend geringere Adhäsionskräfte ausüben.

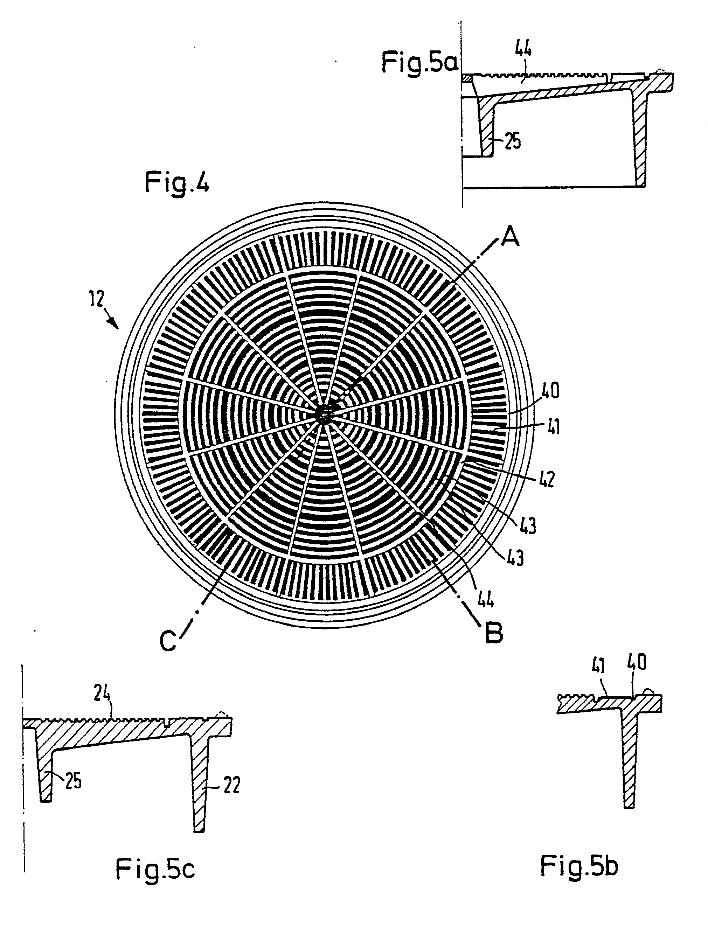

In Fig. 4 ist in Draufsicht ein Behälterunterteil 12 mit dem Dränagesystem und den Tragrippen für die erfindungsgemäß eingesetzten dünnen hydrophilen Membranfilter dargestellt. Die Tragrippen für die Filtermembran 30 sind als volle Linien und die Dränagekanäle als weiße Flächen gekennzeichnet. Wie auch aus den Fig. 5a bis 5c ersichtlich, ist radial außen ein Ringkanal 40 vorgesehen, von dem eine Vielzahl von radialen Stichkanälen 41 ausgehen, die in einen zweiten Ringkanal ausmünden. Im zentralen Bereich sind ferner eine Vielzahl von konzentrischen Ringkanälen 42, 43 angeordnet, die in sternförmige Ablaufkanäle 44 ausmünden, welche gemäß Fig. 5a zum zentralen Ablaufstutzen 25 führen. Die konzentrischen Ringkanäle werden von den Tragrippen 24 für die Filtermembran 30 begrenzt. Der radial äußere Kranz von Radialkanälen hat die angestrebte Wirkung, daß die verschiedenen Behandlungsflüssigkeiten, insbesondere die Prüfflüssigkeit, unmittelbar und auf dem kürzesten Weg aus dem kritischen Randbereich ablaufen können, so daß Ablagerungen von Prüfflüssigkeit in diesen Bereichen auch im Behälterunterteil vermieden werden.

Die erfindungsgemäße Verwendung von durchgehend hydrophilen Filtermembranen 30 hat bei Anwendung der Druckfiltration gegenüber den bisher üblichen Filtermembranen mit hydrophoben Randbereichen noch folgenden Vorteil : Da die hydrophilen Filtermaterialien bis zu einem gewissen Überdruck, der bei den infrage kommenden mikroporösen Filtermaterialien bei ca. 2, 3 bar liegt, gasundurchlässig sind, kann nach Durchlaufen einer Flüssigkeitsmenge im Innenraum der Behälteroberteile 11 oberhalb der Filtermembran ein Luftdruck von ca. 2 bar eingehalten werden. Beim Einfüllen beispielsweise einer Spülflüssigkeit muß daher in den Behälterinnenräumen nicht mehr ein Überdruck erst aufgebaut werden, sondern die Spül- bzw. Füllvorgänge können von Anfang an unter dem gewünschten Betriebsdruck ablaufen. Hierdurch verkürzen sich die Prüfzeiten erheblich.

Die Erfindung ist nicht auf die dargestellten Ausführungen beschränkt. So können beispielsweise die Filtermembranen 30 vorab kreisscheibenförmig zugeschnitten und einzeln von Hand oder maschinell auf die Behälterunterteile aufgelegt werden. Ferner braucht der im Querschnitt keilförmige Ringwulst 35 nicht an der Unterfläche des Behälteroberteils, sondern er kann auch an der Oberfläche des Ringwulstes 27 in der notwendigen Anordnung und Ausbildung angeformt sein, wie dies gestrichelt in den Fig. 5a bis 5c angedeutet ist. Schließlich kann der Träger für die Filtermembran 30 auch als gesondertes scheibenförmiges Bauteil mit Tragrippen und Dränagekanälen ausgebildet sein, das in den Behälterunterteil eingesetzt wird.

## Patentansprüche

1. Vorrichtung zur Sterilitätsprüfung von Fluiden, insbesondere von Antibiotika enthaltenden Flüssigkeiten, mit mindestens einem hermetisch geschlossenen Prüfbehälter aus transparentem Kunststoff, der über Schlauchverbindungen und eine Kanüle steril an ein Probengefäß und an weitere Flüssigkeitsgefäße anschließbar ist, wobei jeder Prüfbehälter aus einem einstückigen Oberteil mit einem Einlaßstutzen, einem Lufteinlaß und einem darin integrierten Luftfilter sowie aus einem einstückigen Unterteil mit einem Dränageglied, einem mechanischen Träger für einen ebenen, mikroporösen Prüffilter und einen Auslaßstutzen besteht, und wobei beide Behälterteile mit den Anschlußschläuchen sowie miteinander druckdicht verschweißt sind und der Prüffilter zwischen dem unteren Rand des zylindrischen Oberteils und dem oberen Rand des zylindrischen Unterteils unlösbar und gasdicht befestigt ist, dadurch gekennzeichnet, daß der gesamte Prüffilter (30) aus einer hydrophilen Filtermembran besteht, und daß jede der beiden Oberflächen des Filtermembran-Randes (33) mit dem jeweiligen Behälterteil (11 bzw. 12) durch eine gesonderte Schweißverbindung (34, 37) druckdicht verbunden ist, die mit der Innenfläche (36) der Behälterwand (20) bündig abschließt.

2. Vorrichtung nach Anspruch 1, dadurch gekennzeichnet, daß die eine Oberfläche des Membranrandes mit dem zugeordneten Rand (27) des einen Behälterteils (12) durch direkte Wärme- und Druckeinwirkung formschlüssig verbunden ist, und daß die andere Oberfläche des Membranrandes (33) mit dem anderen Behälterteil (11) durch einen von radial außerhalb der Filtermembran (30) in den Spalt zwischen der Membran-Oberfläche und der Behälterteilfläche eingedrungenen Schmelzfluß an Behältermaterial verbunden ist.

3. Vorrichtung nach Anspruch 3, dadurch gekennzeichnet, daß an der Stirnfläche eines Behälterrandes eine keilförmige Ringwulst (35) radial außerhalb des Filtermembran-Randes (33) angeformt ist, die beim zweiten Schweißvorgang den Schmelzfluß in den Spalt liefert.

4. Vorrichtung nach einem der Ansprüche 1 bis 3, dadurch gekennzeichnet, daß die durchgehend hydrophile Filtermembran aus einem unverstärkten Zellulosefasermaterial mit einer Dicke von 150 μm besteht.

5. Vorrichtung nach einem der Ansprüche 1 bis 4, dadurch gekennzeichnet, daß das Dränageglied im Behälterunterteil (12) aus einem Ringkanal (40) unmittelbar an der Innenwand, aus einem Kranz von davon ausgehenden Radialkanälen (41) sowie aus Ringkanälen (42, 43) im Innenbereich besteht, wobei diese Dränage-Kanäle (41 bis 43) in sternförmige Ablaufkanäle (44) ausmünden und zwischen den Tragrippen (24) für die Filtermembran (30) angeordnet sind.

6. Verfahren zur Herstellung eines Behälters nach einem der Ansprüche 1 bis 5, bei dem der Behälteroberteil und der Behälterunterteil gesondert in Spritzgußtechnik aus einem durchsichtigen Kunststoff geformt, der Luftfilter in den Oberteil eingeschweißt und der Prüfmembranfilter zwischen den beiden Behälterteilen befestigt wird, dadurch gekennzeichnet, daß die Filtermembran über dem Rand eines Behälterteils positioniert und ihre Oberfläche durch Zufuhr von Wärme duch die Filtermembrane hindurch unter einem vorbestimmten Druck mit dem Behälterrand verschweißt bzw. verklebt wird, und daß anschließend der zweite Behälterteil mit seinem Rand mit der anderen Oberfläche der Membran und gleichzeitig mit dem ersten Behälterteil druckdicht verschweißt wird.

7. Verfahren nach Anspruch 6, dadurch gekennzeichnet, daß zur Herstellung der zweiten Schweißverbindung eine an einem der Behälterteile angeformte Ringwulst erhitzt und aufgeschmolzen wird, wobei der schmelzflüssige Kunststoff beim Zusammenbringen beider Behälterteile den Spalt zwischen der Oberfläche des Filtermembranrandes und dem Behälterteil vollständig ausfüllt.

8. Verfahren nach Anspruch 6 oder 7, dadurch gekennzeichnet, daß das mikroporöse Filtermaterial bandförmig von einer Rolle taktweise bis über die positionierten ersten Behälterteile abgezogen wird, daß aus dem Bandmaterial scheibenförmige Filterblätter ausgestanzt werden, und daß jede Oberfläche des Filtermembranrandes in einem gesonderten Schweißvorgang mit dem jeweiligen Behälterteil druckdicht und dauerhaft verbunden wird.

9. Verfahren nach Anspruch 8, dadurch gekennzeichnet, daß das Ausstanzen der scheibenförmigen Filtermembranen nach dem ersten Schweißvorgang erfolgt.

## Claims

1. Device for testing the sterility of fluids, especially antibiotics-containing liquids, comprising at least one hermetically sealed test container of a transparent plastics material, said container being adapted to be connected in a sterile manner to a sample vessel and to further liquid vessels through hose connections and a canula, with each test container comprising an integral upper portion including an inlet port, an air inlet and an air filter integrated in the latter, as well as an integral lower portion including a draining member, a mechanical support for a planar microporous test filter and an outlet port, and with the two container portions being pressure-tightly welded or sealed to the connecting hoses and to each other, and the test filter being mounted undetachably and gas-tightly between the lower rim of the cylindrical upper portion and the upper rim of the cylindrical lower portion, characterized in that the complete test filter (30) comprises a hydrophilic filter membrane (or diaphragm), and that either of the two surfaces of the filter membrane rim (33) is pressure-tightly bonded to the respective container portion (11 or 12) by a separate welding joint (34, 37) which is flush with the inner surface (36) of the container wall (20).

2. The device according to claim 1, characterized in that the one surface of the (peripheral) rim of the membrane is joined with positive engagement to the associated rim (27) of the one container portion (12) by direct heat and pressure action, and that the other surface of the rim (33) of the membrane is joined or bonded to the other container portion (11) by a fused mass of container material penetrated from radially outside of the filter membrane (30) into the gap between the membrane surface and the container portion surface.

3. The device according to claim 3, characterized in that the end face of a container rim has formed thereon a wedge-shaped torus (35) radially outside of the filter membrane rim (33), which torus provides for the fused mass in the gap during the second welding operation.

4. The device according to any one of claims 1 to 3, characterized in that the continuously hydrophilic filter membrane comprises a non-reinforced cellulose fibrous material having a thickness of 150 μm.

5. The device according to any one of claims 1 to 4, characterized in that the draining member in the lower portion (12) of the container comprises an annular groove (40) directly at the inner wall, a ring of radial grooves (41) extending from the annular groove, as well as annular grooves (42, 43) in the inner region, with these draining grooves (or channels) (41 to 43) terminating in star-shaped drain channels (44) and being disposed between the supporting ribs (24) for the filter membrane (30).

6. A method of producing a container according to any one of claims 1 to 5, wherein the upper portion and the lower portion of the container are molded separately from a transparent plastics material by injection molding, the air filter is welded or sealed into the upper portion, and the test membrane filter is secured between said two container portions, characterized in that the filter membrane is positioned above the (peripheral) rim of one container portion and has its surface welded or bonded to the container rim by applying heat through the filter membrane, under a predetermined pressure ; and that subsequently the second container portion has its rim welded in pressure-tight manner to the other surface of the membrane and simultaneously to the first container portion.

7. The method according to claim 6, characterized in that for producing the second welded joint, a torus formed on one of the container portions is heated and molten, wherein the liquid molten plastics material fills out completely the gap between the surface of the filter membrane rim and the container portion when the two container portions are being brought together.

8. The method according to claims 6 or 7, characterized in that the microporous filter material is cyclically withdrawn in a web shape from a coil to a position above the positioned first container portions ; that disc-shaped filter sheets are punched out from the web material ; and that each surface of the (peripheral) rim of the filter membrane is bonded pressure-tightly and permanently to the respective container portion in a separate welding step.

9. The method according to claim 8, characterized in that punching out of the disc-shaped filter membranes is effected after the first welding operation.

## Revendications

1. Dispositif pour contrôler la stérilité de fluides, notamment de liquides contenant des antibiotiques, comportant au moins un récipient d'essai fermé de façon hermétique et constitué d'une matière plastique transparente et qui peut être raccordé de façon stérile, par l'intermédiaire de liaisons en forme de tuyaux et d'une canule, à un récipient d'échantillonnage et à d'autres récipients pour liquides, et dans lequel chaque récipient de contrôle est constitué par une partie supérieure réalisée d'un seul tenant et comportant une tubulure d'admission, une admission pour l'air et un filtre à air, intégré dans cette admission, ainsi que par une partie inférieure réalisée d'un seul tenant et comportant un élément de drainage, un support mécanique pour un filtre de contrôle microporeux et une tubulure de sortie, et dans lequel les deux parties du récipient sont soudées, d'une façon étanche à la pression, aux tuyaux de raccordement ainsi qu'entre eux, et le filtre de contrôle est fixé, de façon imperdable et d'une manière étanche au gaz, entre le bord inférieur de la partie supérieure cylindrique et le bord supérieur de la partie inférieure cylindrique, caractérisé en ce que l'ensemble du filtre de contrôle (30) est constitué par une membrane de filtre hydrophyle et que chacune des deux surfaces du bord (33) de la membrane formant fil est reliée, d'une façon étanche à la pression, à la partie respective (11 ou 12) du récipient par une liaison soudée particulière (34, 37) qui se termine de niveau avec la surface intérieure (36) de la paroi (20) du récipient.

2. Dispositif selon la revendication 1, caractérisée en ce qu'une surface du bord de la membrane est reliée selon une liaison par formes complémentaires au bord associé (27) d'une partie (12) du récipient, au moyen de l'action directe d'une chaleur et d'une pression, et que l'autre surface du bord (33) de la membrane est reliée à l'autre partie (11) du récipient au moyen d'une masse fondue du matériau du récipient, introduite depuis l'extérieur, du point de vue radial, de la membrane de filtre (30) dans la fente comprise entre la surface de la membrane et la surface de la partie du récipient.

3. Dispositif selon la revendication 2, caractérisé en ce qu'un bourrelet annulaire en forme de coin (35), qui délivre la masse fondue dans la fente lors de la seconde opération de soudure, est réalisé par façonnage sur la surface frontale d'un bord du récipient, à partir de l'extérieur, du point de vue radial, du bord (33) de la membrane du filtre.

4. Dispositif selon l'une des revendications 1 à 3, caractérisé en ce que la membrane de filtre totalement hydrophyle est constituée par un matériau non renforcé formé de fibres de cellulose et possédant une épaisseur de 150 μm.

5. Dispositif selon l'une des revendications 1 à 4, caractérisé en ce que l'élément de drainage situé dans la partie inférieure (12) du récipient est constitué par un canal annulaire (40) situé directement contre la paroi intérieure et par une couronne de canaux radiaux (41) partant de ce canal annulaire, ainsi que par des canaux radiaux (42, 43) situés dans la zone intérieure, auquel cas ces canaux de drainage (41 à 43) débouchent dans des canaux d'évacuation (44) disposés en étoile et sont disposés entre les nervures de support (24) pour la membrane de filtre (30).

6. Procédé pour fabriquer un récipient selon l'une des revendications 1 à 5, selon lequel on forme la partie supérieure et la partie inférieure du récipient séparément selon la technique de moulage par injection au moyen d'une matière plastique transparente, on soude le filtre à air dans la partie supérieure et on fixe le filtre à la membrane de contrôle entre les deux parties du récipient, caractérisé en ce qu'on positionne la membrane du filtre sur le bord d'une partie du récipient et qu'on soude ou on colle sa surface sur le bord du récipient, par application d'une chaleur à travers la membrane du filtre et sous une pression prédéterminée, et qu'ensuite on soude, d'une manière étanche au gaz, la seconde partie du récipient par son bord sur l'autre surface de la membrane et simultanément sur la première partie du récipient.

7. Procédé selon la revendication 6, caractérisé en ce que, pour réaliser la seconde liaison soudée, on chauffe et on fait fondre un rebord annulaire réalisé par façonnage sur l'une des parties du récipient, auquel cas la matière plastique fondue liquide remplit complètement la fente située entre la surface du bord de la membrane de filtre et la partie du récipient, lors de la réunion des deux parties du récipient.

8. Procédé selon la revendication 6 ou 7, caractérisé en ce qu'on retire d'un rouleau, de façon cadencée sous la forme d'une bande, le matériau de filtre microporeux en l'amenant jusqu'à les premières parties positionnées du récipient, qu'on découpe des feuilles de filtre en forme de disques à partir du matériau en forme de bande et qu'on relie, d'une manière étanche à la pression et de façon durable, chaque surface du bord de la membrane de filtre à la partie respective du récipient, au cours d'une opération distincte de soudage.

9. Procédé selon la revendication 8, caractérisé en ce que le découpage des membranes de filtre en forme de disques d'effectue après la première opération de soudage.

Fig.1

Fig.2a

Fig.2b

Fig.3

0 118 601

Fig.5a

44

25

Fig.4

A

12

40

41

42

43

43

44

C

B

24

25

22

Fig.5c

41 40

Fig.5b